# EUROPEAN PATENT APPLICATION

(11) **EP 1 747 784 A1**
(43) Date of publication of application: **31.01.2007**
(21) Application number: 05740959.1
(22) Date of filing: 17.05.2005
(51) Int. Cl.: A61K 31/711, A61P 35/00, C07K 14/82, C07K 16/32

(54) **TARGET GENE MIMITIN OF MYC**

(30) Priority: 18.05.2004 JP 2004147964
(71) Applicant: KURUME UNIVERSITY, Kurume-shi, Fukuoka 830-0011 (JP)
(72) Inventor: TSUNEOKA, Makoto, Gunma 370-0854 (JP)
(74) Representative: Helbing, Jörg
(86) International application number: PCT/JP2005/009302
(87) International publication number: WO 2005/111213

(57) **Abstract**

A new diagnosis or treatment of cancer is provided. The present invention provides a protein Mimitin which is a target of a cancer gene myc protein, a variant thereof and a fragment thereof, as well as a polynucleotide molecule encoding them. An inhibitory substance of biological activity of the present invention, which is afforded by the binding of a polynucleotide molecule encoding a Mimitin protein and Myc provides a useful means for the diagnosis, prophylaxis or treatment of cancer.

## Description

### Technical Field

The present invention relates to a Myc target gene, mimitin, a protein encoded by said gene or a variant thereof, a production method of said protein, a molecule inhibiting the activity of said protein, a pharmaceutical composition containing said molecule, a method of screening for a substance inhibiting said gene, and diagnosis of cancer utilizing expression of said protein.

### Background Art

It has been long known that the proto-oncogene myc family is particularly related to cancer. It is a cell proliferation-associated gene which plays an important role in cancer, ontogeny and the like. The abnormality of myc gene has been found in human cancer at a very high ratio. The myc family consists mainly of three genes: c-myc, N-myc, and L-myc. Although the three genes exhibit distinct sites of expression and timing, they appear to have basically the same biological activity.

c-myc oncogene is one of the most widely studied proto-oncogenes of the myc gene family. The expression of c-myc is highly regulated by the levels of transcription, after transcription and the like. In general, c-myc expression is associated with cell proliferation and is down-regulated in quiescent and differentiated cells. Deregulated expression of myc family genes through gene proliferation, viral promoter insertion, chromosomal translocation, or promoter mutation is associated with neoplastic diseases (so-called tumorigenesis) in a wide range of vertebrates including humans (DePinho, R. A., et al.: (1991) Adv. Cancer Res. 57, 1-46, Marcu, K. B., et al.: (1992) Annu. Rev. Biochem. 61, 809-860, Morgenbesser, S.D., et al.: (1994) Semin. Cancer Biol. 5, 21-36, Henriksson, M., et al.: (1996) Adv. Cancer Res. 68, 109-182, Grandori, C., et al.: (2000) Annu. Rev. Cell. Dev. Biol. 16, 653-99).

Embryonic mice with c-myc or N-myc deleted develop multi-organ hypoplasia and die during mid-embryogenesis (Stanton, B. R., et al.: (1992) Genes Dev. 6, 2235-2247; Sawai, S., et al.: (1993) Development 117, 1445-1455; Davis, A. C., et al.: (1993) Genes Dev. 7, 671-682). These results indicate that the myc is the central regulator of cell growth (Henriksson, M., et al. (1996) Adv. Cancer Res. 68, 109-182; Grandori, C., et al.: (2000) Annu. Rev. Cell. Dev. Biol. 16, 653-99; Luscher, B.: (2001) Gene 277, 1-14).

These cancer genes, myc family genes, encode the transcription factor (Myc protein) which regulates the expression of various genes (Grandori, C., et al.: (2000) Annu. Rev. Cell. Dev. Biol. 16, 653-99; Luscher, B.: (2001) Gene 277 (Oct 17; (1-2)), 1-14). The protein encoded by myc family gene is a member of the basic-helix-loop-helix leucine zipper (bHLHLZ) transcription factors. Myc protein and Max result in the formation of a heterodimer, which binds to E-box site (mainly CACGTG element) and activates gene transcription. Various Myc target genes, for example, ODC (Bello-Fernandez, C., et al.: (1993) Proc. Natl. Acad. Sci. USA 90, 7804-7808), cyclin D2 (Bouchard, C., et al.: (2000) Genes & Dev 15, 2042-2047), mina53 (Tsuneoka M, et al.: (2002) J. Biol. Chem. 277, 35450-35459) and the like have been identified (Dang, C.V.: (1999) Mol. Cell. Biol. 1-11). However, Myc target genes enough to elucidate the function of cancer gene myc have not been identified yet, and therefore, ghd mechanism for exerting functions of myc gene has not been satisfactorily clarified.

### Disclosure of the Invention

Cancer occupies a large rate of the cause of the human death, and further is apt to increase.

It is considered that, although cancer cells are originally normal cells, when the genomic DNA of normal cells is damaged by certain causes such as chemical substances, radiation or infection with virus and the like and the damaged DNA is not restored but accumulated in the normal cell, it induces the normal cells to turn cancerous. Various cancer-associated genes have been found hereinbefore and there include cancer genes which accelerate cancer and cancer-inhibitory genes which suppress cancer.

It has been known that the proto-oncogene myc family as one family of such cancer-associated genes is particularly related to cancer. Such family is cell proliferation-associated gene which plays an important role in cancer and ontogeny and the like, and the abnormality of myc gene has been found in human cancer at a very high ratio. Accordingly, clarification of the function of the Myc protein encoded by the gene, for example, identification of the new genes expressed and regulated by Myc protein can help elucidate the function of cancer gene myc.

The number of patients suffering from esophageal squamous cell carcinoma has been increasing in Japan, and the number shows a steep increase with aging. At present, the number of patients who die of esophageal cancer amounts to about 3% of the total number of patients who die of cancer. Esophageal cancer is an aggressive disease with a poor prognosis. In the forthcoming aging society, there is concern that the number of esophageal cancer patients may further increase, and esophageal cancer is becoming an important disease for the health of Japanese people. As a result of the study for elucidating an esophageal cancer-related gene, it has been shown that a functional loss of tumor suppressor gene and activation or abnormal expression of cancer gene are responsible for oncogenesis, where abnormal expression of c-myc has also been reported (Kennedy AR: Cancer Res. 1994, 54 (7 Suppl) : 1999s-2005s).

Thus, the present inventors have searched for a cancer-associated gene that can be a novel cancer marker, and found a gene, mimitin, which is a new target for c-Myc protein and a protein mimitin encoded by the gene.

Accordingly, the present invention relates to
(1) a protein having an amino acid sequence shown in SEQ ID NO: 1 or SEQ ID NO: 3, or a variant of said protein, which has an amino acid sequence shown in SEQ ID NO: 1 or SEQ ID NO: 3, wherein one to several amino acid residues are at least substituted, deleted, inserted or added, and has substantially the same level of activity as the cell proliferation activity of said protein, or a fragment thereof;
(2) an antibody against the protein of the above-mentioned (1) or a variant thereof;
(3) the polynucleotide molecule encoding the protein of the above-mentioned (1) or a variant thereof, specifically, a polynucleotide molecule which is a DNA molecule, or a polynucleotide molecule located in the vicinity of human chromosome 5q12, which corresponds to a gene showing upregulated transcription due to binding of c-Myc protein, more specifically, a polynucleotide molecule having the 128th - 634th position of SEQ ID NO: 2, or the base sequence shown in SEQ ID NO: 4, preferably, a polynucleotide molecule encoding a protein having the base sequence shown in SEQ ID NO: 2 and cell proliferation activity, or a polynucleotide molecule having the base sequence shown in SEQ ID NO: 4;
(4) a vector containing the polynucleotide molecule of the above-mentioned (3);
(5) a transformant obtained by introducing the vector of the above-mentioned (4) into a host cell;
(6) a method of producing the protein of the above-mentioned (1), which comprises culturing the transformant of the above-mentioned (5) and recovering the resulting protein from a culture;
(7) a polynucleotide or oligonucleotide molecule having a sequence complementary to a full length or a part of the base sequence shown in SEQ ID NO: 2 or SEQ ID NO: 4, or a variant thereof, which is a molecule inhibiting the cell proliferation activity of the protein of the above-mentioned (1), specifically, an inhibitory molecule which is an antisense oligonucleotide molecule, or a small interfering RNA (siRNA) molecule containing a part of the base sequence shown in SEQ ID NO: 2 or SEQ ID NO: 4, and a complementary chain thereof, which specifically inhibits gene expression;
(8) a pharmaceutical composition containing the molecule of the above-mentioned (7) as an active ingredient, preferably a pharmaceutical composition for the prophylaxis or treatment of cancer;
(9) a diagnostic agent for cancer comprising the antibody of the above-mentioned (2);
(10) a method of detecting the protein of the above-mentioned (1) in a patient suspected of having a cancer, which comprises labeling a tissue obtained from the patient with the antibody of the above-mentioned (2), and judging the level of labeling;
(11) a method of screening for a substance inhibiting the cell proliferation activity of the polynucleotide molecule of the above-mentioned (3), which comprises adding a sample containing a candidate inhibitory substance to a reaction system containing the polynucleotide molecule and a c-Myc protein, and determining the activity of the candidate substance to inhibit binding of the polynucleotide molecule and the c-Myc protein;
(12) a method for the prophylaxis or treatment of cancer, which comprises administering the pharmaceutical composition of the above-mentioned (8) to an animal; and
(13) a commercial package comprising the pharmaceutical composition of the above-mentioned (8) and a written matter associated therewith, the written matter stating that the pharmaceutical composition can or should be used for preventing or treating cancer.

The present invention is based on a Myc target gene, mimitin, whose expression product is mainly found in mitochondria, and which shows upregulated expression directly caused by a c-Myc protein.

Mimitin is a novel c-Myc target gene, which is found to be necessary for the proliferation of esophageal cancer cells. Hence, the mimitin gene is considered to be applicable to the diagnosis or treatment of cancer such as esophageal cancer and the like. An inhibitor (inhibitory substance) that inhibits the action of the mimitin gene is potentially a novel anti-cancer agent.

### Brief Description of the Drawings

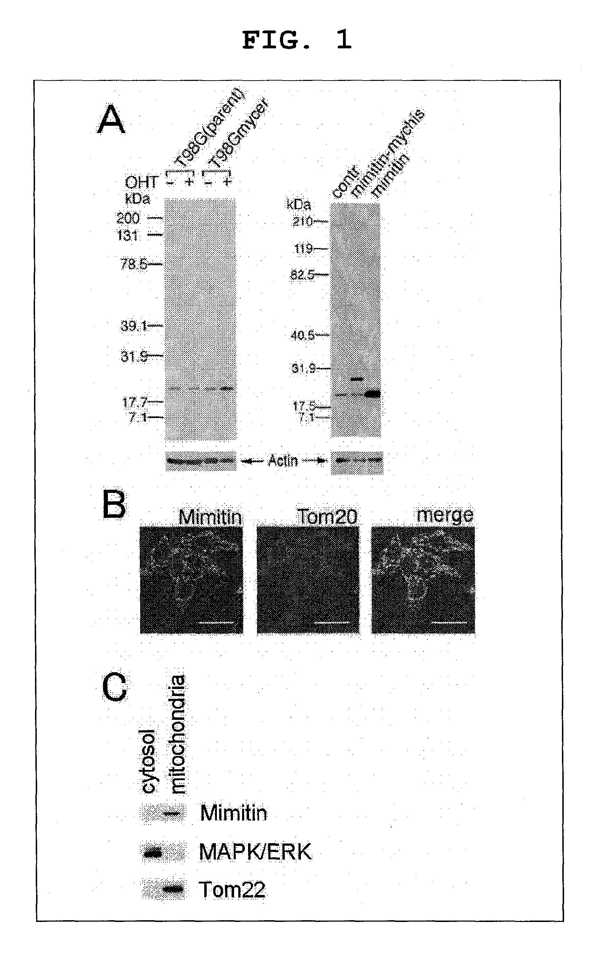

Fig. 1 is a photograph instead of a drawing, which shows the intracellular localization of the Mimitin protein. Fig. 1A shows a Western blotting analysis. The left panel shows that an anti-Mimitin antibody specifically recognizes Mimitin in T98G cells, and the Mimitin protein increases by activation of MycER protein in T98Gmycer cells with OHT, and the right panel shows detection of Mimitin in T98G cells incorporating a Mimitin expression vector (mimitin or mimitin-mychis). Fig. 1B shows localization of Mimitin in mitochondria. Fig. 1C shows that Mimitin is fractionated into a mitochondrial fraction.

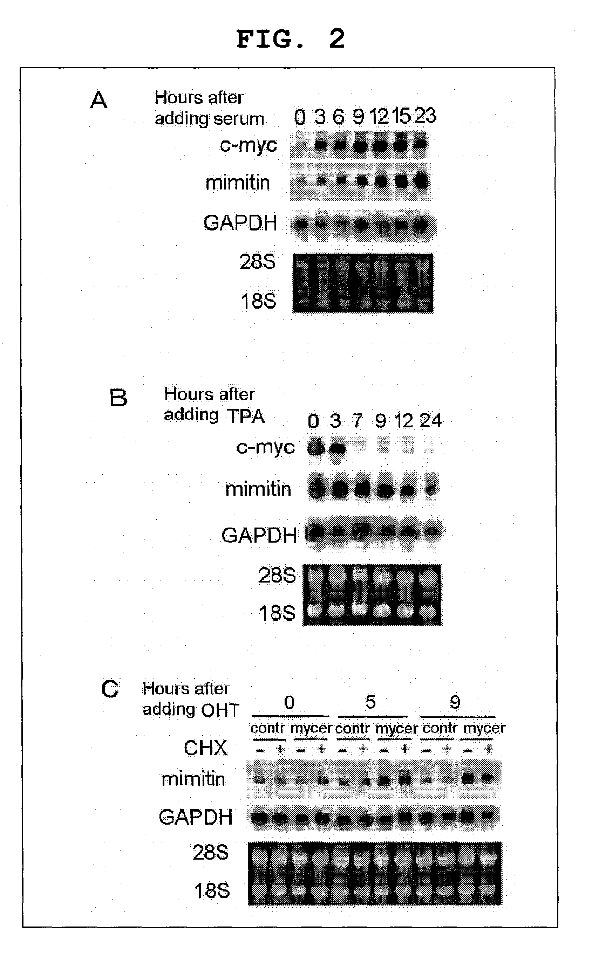

Fig. 2 is a photograph instead of a drawing, which shows the expression level of mimitin mRNA. Fig. 2A shows that the expression level of mimitin mRNA is associated with the c-myc expression level in T98G cells. Fig. 2B shows the relationship between the lower expression level of mimitin mRNA and disappearance of c-myc mRNA in human promyelocytic leukemia HL60 cells. Fig. 2C shows expression induction of mimitin mRNA in T98Gmycer-2 cells where MycER protein was activated with 4-hydroxytamoxifen (OHT) (mycer in the Figure) and that the expression induction is not influenced by cycloheximide (CHX).

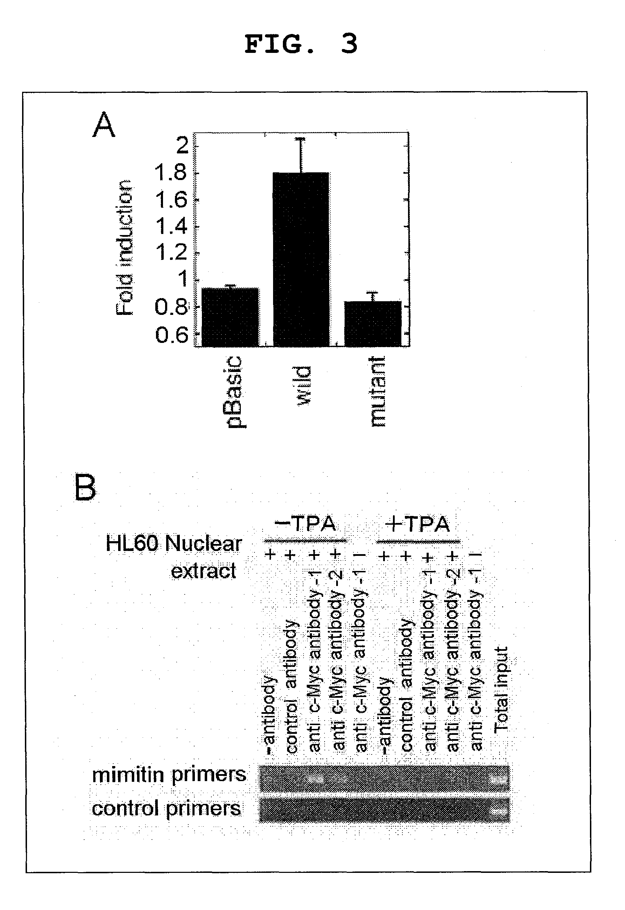

Fig. 3 is a photograph instead of a drawing, which shows that c-Myc is directly bound to mimitin genome gene to increase its expression. Fig. 3A shows the results of a transient expression assay of reporter plasmid where the mimitin promoter activity was determined using T98Gmycer-2 cells. Fig. 3B shows the experiment results of chromatin immunoprecipitation using HL60 cells.

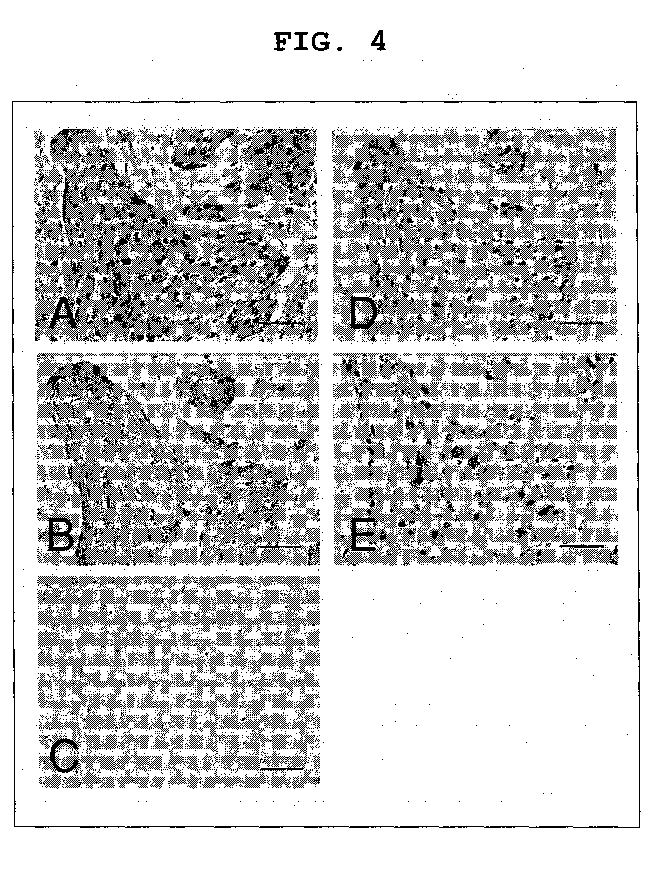

Fig. 4 is a photograph of the cell morphology showing the presence of Mimitin in esophageal cancer. In each photograph, a bar shows a 50 µm scale. Fig. 4A shows HE staining, Fig. 4B shows immunostaining using anti-Mimitin antibody, Fig. 4C shows immunostaining using no primary antibody, Fig. 4D shows immunostaining using anti-c-Myc antibody, and Fig. 4E shows immunostaining using anti-Ki-67 antibody.

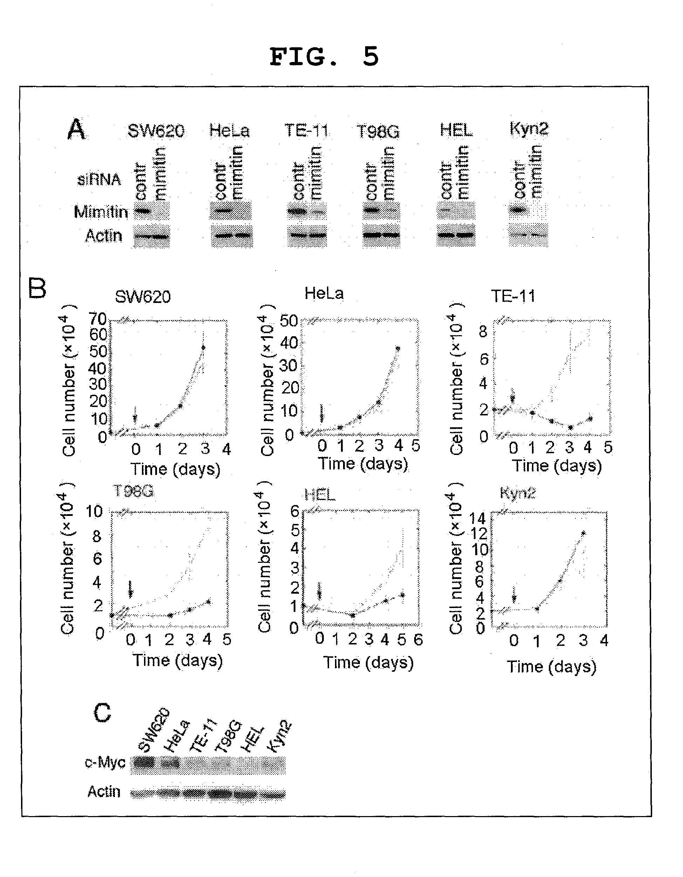

Fig. 5 shows involvement of Mimitin in cell proliferation. Fig. 5A is a photograph instead of a drawing, which shows that mimitin specific siRNA inhibits Mimitin expression. Fig. 5B shows changes in the cell number of various cells incorporating mimitin specific siRNA. In the Figure, ● shows cells incorporating si-mimitin-2 RNA, ○ shows cells incorporating control siRNA and ↓ shows the date of addition of serum. Fig. 5C is a photograph instead of a drawing, which shows expression of c-Myc.

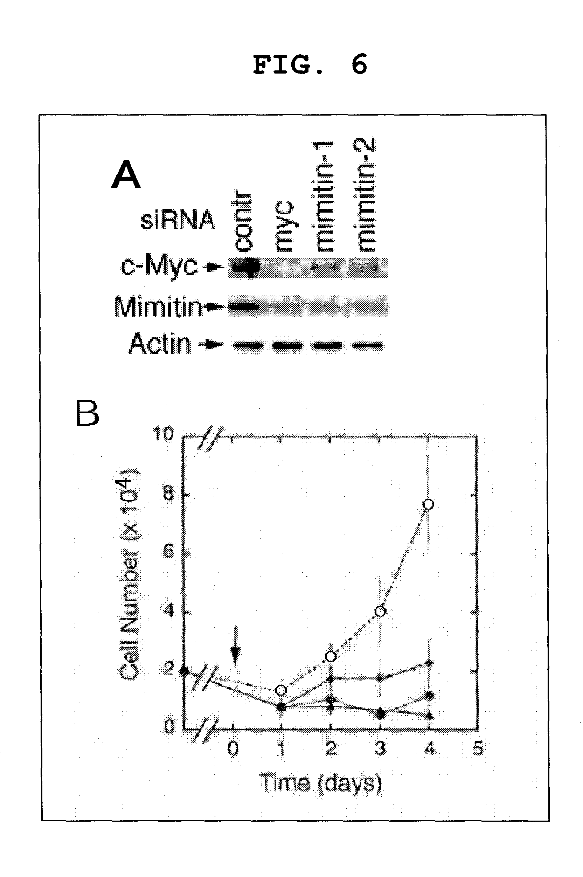

Fig. 6 shows that mimitin specific siRNA inhibits cell proliferation. Fig. 6A is a photograph replacing drawing. Fig. 6B shows cell number in a graph, wherein ◆ shows cells incorporating si-mimitin-1 RNA, ▲ shows cells incorporating si-mimitin-2 RNA, ● shows cells incorporating c-myc RNA and ○ shows cells incorporating control siRNA, and ↓ shows the date of addition of serum.

### Best Mode for Embodying the Invention

The gene, mimitin, (Myc-induced mitochondrial protein) of the present invention encodes a protein with a molecular mass of about 20 kDa, which localizes in mitochondria. Transcription of mimitin gene is directly stimulated by Myc. Myc in the present invention encompasses c-Myc, N-Myc and L-Myc, and is not particularly limited. However, the explanation focuses on c-Myc for which gene amplification, overexpression and translocation have been reported in various cancers. The expression of Mimitin protein in esophageal cancer generally said to show poor prognosis was examined.
As a result, it was clarified that Mimitin protein was highly expressed in almost all esophageal cancer samples. This suggests that expression of Mimitin protein can be an index for detection of esophageal cancer. Comparison of the level of cell proliferation, level of tumor differentiation, level of tumor progression, and level of Mimitin staining revealed no relationship between tumor differentiation level, tumor progression level and Mimitin staining level. However, there was a clear correlation between the Mimitin staining level and cell proliferation. It was further clarified that specific inhibition of mimitin expression by RNA interference method resulted in the suppressed proliferation of cultured cancer cells. These indicate that novel Myc target gene, mimitin, encodes a protein localized in mitochondria, which protein being involved in the growth of cells of cancer such as esophageal cancer.

More particularly, the present invention relates to a protein having an amino acid sequence shown in SEQ ID NO: 1 or SEQ ID NO: 3 (hereinafter to be referred to as Mimitin protein), or a variant of said protein, which has an amino acid sequence shown in SEQ ID NO: 1 or SEQ ID NO: 3, wherein one to several amino acid residues are at least substituted, deleted, inserted or added, and has substantially the same level of activity as the cell proliferation activity of said protein, or a fragment thereof.

The protein of the present invention can be prepared by a conventional method such as genetic engineering, protein synthesis and the like. The outline is explained in the following.

### (1) Cloning of c-Myc target gene, mimitin

A DNA fragment containing a DNA encoding the Mimitin protein of the present invention is isolated by the methods explained in detail in the following Examples.

By a brief explanation, the expression of the cancer-associated gene, mimitin, of the present invention is directly induced by a cancer gene, c-myc. Utilizing this, for isolation of mimitin gene, a cell line having an artificially inducible c-Myc activity is prepared. For this purpose, for example, an estrogen-inducible Myc system (Eilers, M., et al.: (1989) Nature 340, 66-68) can be used. Chimeric protein c-MycER consists of human c-Myc and an estrogen binding domain of a human estrogen receptor. The chimeric protein binds to a cytoskeleton component in the absence of estrogen. When the chimeric protein is bound to estrogen or its homolog, 4-hydroxytamoxifen (OHT), however, it is liberated from the cytoskeleton component and functions as c-Myc.

A plasmid encoding c-Myc to be used for preparing chimeric protein c-MycER can be produced by a conventional method in the art. As the plasmid to be used, various plasmids are commercially available, which can be appropriately selected according to the object of use. For example, pc-mycer/CAGGS prepared by inserting c-mycer constructed from Pc-myc/CDM8 (Tsuneoka M., Nakano F., Ohgusu H., and Mekada E., c-myc activates RCC1 gene expression through E-box elements, Oncogene, 14: 2301-2311, 1997) that induces expression of human c-Myc by CMV promoter, and a plasmid encoding an estrogen binding domain, such as pBSKSR-ER (Tsuneoka M., Nakano F., Ohgusu H., and Mekada E., c-myc activates RCC1 gene expression through E-box elements, Oncogene, 14: 2301-2311, 1997), into the downstream of a chimera promoter constructed from a chicken-actin promoter and a CMV promoter, and the like can be used (Tsuneoka M., Koda Y., Soejima M., Teye K. Kimura H. A novel Myc target gene, mina53, that is involved in cell proliferation. J. Biol. Chem. 277 (38): 35450-35459, 2002). According to a conventional method, they are transfected into a cell such as human glioblastoma cell line T98G cells (human, Caucasian, glioblastoma, ECACC 92090213, ATCC CRL 1690) and the like.

Then, a gene showing enhanced expression due to c-Myc activity is searched for by differential display. With regard to the obtained cells, RNA is isolated from each of the untreated cells and the cells where the c-Myc activity has been induced, the expression of RNA is examined by cDNA microarray and the like and a gene stimulated by c-MycER activation to show enhanced expression is identified.

Based on the sequence information of the identified gene, a full-length cDNA is prepared by RACE system, RT-PCR and the like and the sequence is determined. The thus-obtained cancer-associated gene, mimitin, of the present invention has the base sequence shown in SEQ ID NO: 2 (128th - 634th) for human and the base sequence shown in SEQ ID NO: 4 for mouse. Other ortholog can also be obtained in the same manner as above. Alternatively, an ortholog can also be obtained by screening cDNA libraries derived from various animals using a probe designed based on a human base sequence (SEQ ID NO: 2). As animals other than human and mouse, rat, guinea pig, hamster, rabbit, swine, bovine, horse, sheep, goat, dog, cat, bird, monkey and the like can be mentioned.

Preferred is a human or mouse mimitin gene. Human mimitin gene consists of 4 exons, and human mimitin gene is mapped to chromosome 5 (5q12.1) in the UCSC Genome Browser mapping data.

### (2) Expression of recombinant protein

The obtained cDNA is then incorporated into a suitable expression vector and the vector is used as an expression vector for expressing a Mimitin protein. As an appropriate expression vector, for example, pET28 (Novagen, Madison, WI, Cat# 69865-3) and pGEX-3X (GenBank data base, accession No. U13852) can be used for bacterium, and pCAGGS (Niwa H., Yamamura K., and Miyazaki J., (1991), Gene, vol. 108, 193-199)), pRC/CMV (Invitrogen) and pcDNA3.1/MycHis(-) B (Invitrogen Cat# V855-20) can be used for animal cells.
Besides these, vectors for yeast and vectors used for insect cells can also be used. The expression vector is introduced into a suitable host cell, such as bacterium, yeast, insect cell, or animal cell to prepare a transformant.

Alternatively, a Mimitin protein can be totally synthesized using, for example, a solid phase method and a known, already-reported method (Clark-Lewis et al., Biochemistry 30:3128-3135, 1991).

The obtained protein can be purified by a combination of methods well known to those of ordinary skill in the art, for example, affinity column, ion exchange chromatography, gel filtration chromatography, reversed-phase chromatography, hydrophobic chromatography, and the like.

The obtained Mimitin protein shows the following characteristics:
A) its expression is directly enhanced by c-Myc;
B) it is primarily localized in mitochondria;
C) staining of esophageal cancer tissue with an anti-Mimitin antibody affords a well-stained cancer part. This indicates that the Mimitin protein is a cancer marker;
D) the staining pattern is quite similar to that of anti-Ki-67 antibody. Ki-67 is expressed only in the cells under proliferation, and is generally used for finding a cell under proliferation in a tissue. When a cell stained with an anti-Mimitin antibody is also stained with an anti-Ki-67 antibody, it means that a cell in which Mimitin is expressed is growing;
E) when expression of Mimitin was inhibited in the culture cell of esophageal cancer, the cell proliferation was inhibited. When expression of c-Myc was inhibited in the same cell, the cell proliferation was inhibited again, and the expression of Mimitin was also inhibited. Therefrom, it is considered that c-Myc positively controls the cell proliferation in this cell, and enhancement of the Mimitin expression is involved in the control.

A variant of the protein of the present invention is expressed using a DNA mutated by a genetic recombination technique (Sambrook et al., Molecular Cloning: A laboraroy manual, 2nd edn. New York, Cold Spring Harbor Laboratory) well known to those of ordinary skill in the art.

The activity of substantially the same level as the cell proliferation activity of the variant of the present invention in Mimitin protein is determined as follows.

When a mimitin expression vector (pCAGGS/mimitin) was introduced into an esophageal cancer cell, the Mimitin protein was strongly expressed in the cell. It was observed then that the amount of a cell proliferation marker protein (protein that increases when cell proliferation is high, which is detected, for example, by phosphorylated Histone H3,
Antiphospho-Histone H3 (Upstate Cat# 06-570)) increased. This means that the Mimitin protein is involved in cell proliferation. Therefore, the activity of substantially the same level as the cell proliferation activity of the variant of the present invention in Mimitin protein can be judged with the increase level of the cell proliferation marker protein as an index.

The variant of the present invention preferably shows the following biological activity as in Mimitin protein:
A) its expression is directly enhanced by c-Myc;
B) it is primarily located in mitochondria;
C) staining of esophageal cancer tissue with an anti-variant antibody affords a well-stained cancer part.
D) the staining pattern is quite similar to that of anti-Ki-67 antibody.

A fragment of the Mimitin protein or a variant thereof preferably shows the same biological activity as in Mimitin protein, like the variant of the present invention.

As another embodiment, the present invention relates to an antibody against the protein of the present invention or a variant thereof, particularly a monoclonal antibody, and a hybridoma cell that produces the monoclonal antibody. The antibody can be produced according to a conventional method usually employed in said technical art. The monoclonal antibody of the present invention can be prepared using a hybridoma prepared by fusion methods such as a cell fusion method using polyethylene glycol, an electric fusion method and the like, a method using Sendai virus, Epstein-Barr virus and the like, a method using a recombinant by gene recombination, a transformant by a tumor promoter and the like, and the like (Koehler, G., Milstein, C.: Nature 256, 495 (1975)). Particularly, establishment of hybridoma by a cell fusion method using polyethylene glycol is preferable.

The antibody of the present invention can be preferably used for diagnosing diseases involving Mimitin, specifically cancer.

As another embodiment, the present invention relates to a polynucleotide molecule encoding the Mimitin protein of the present invention or a variant of said protein.

In the present specification, the "polynucleotide molecule" also encompasses DNA and RNA. In the case of DNA, it can be cDNA, genomic DNA or synthetic DNA. DNA and RNA may be a double strand or a single strand. In the case of single strand, it can be coding strand or non-coding strand. RNA also encompasses siRNA (small interfering RNA).

To be specific, it relates to a polynucleotide molecule containing the base sequence shown in the 128th - 634th of SEQ ID NO: 2, or the base sequence shown in SEQ ID NO: 4, or a variant thereof. As used herein, the variant means a variant produced by the base substitution, base addition or allele mutation of the above-mentioned polynucleotide molecule. The "variant produced by allele mutation" means a base mutation based on individual difference or race difference, which is naturally present, including changes in the amino acid sequence to be encoded.

A preferable embodiment is a polynucleotide molecule encoding human Mimitin having a cell proliferation activity, which has the base sequence shown in SEQ ID NO: 2, or a polynucleotide molecule having the base sequence shown in SEQ ID NO: 4.

The present invention moreover relates to an inhibitory molecule, which is a polynucleotide or oligonucleotide molecule having a sequence complementary to a full or partial base sequence shown in SEQ ID NO: 2 or SEQ ID NO: 4, or a variant thereof, which is a molecule inhibiting the cell proliferation activity of the protein of the present invention.

Typically, an antisense oligonucleotide having a sequence complementary to 128th - 634th of SEQ ID NO: 2, or the base sequence of SEQ ID NO: 4 is included.

In SEQ ID NO: 2, the inhibitory molecule may contain a complementary chain to the 5' or 3' non-coding region. Particularly, a sequence complementary to the 5' non-coding region can be mentioned, which includes a molecule having a sequence complementary to a transcription initiation site, a translation initiation site, a 5' non-translation region and the like. A preferable length of the inhibitory molecule is about 15 base pairs (bp) to about 100 bp.

An inhibitory molecule relating to the base sequence shown in SEQ ID NO: 2 is preferable.

The inhibitory molecule particularly includes a small interfering RNA (siRNA). A recent report has documented that a siRNA double-stranded chain consisting of 21 nucleotides specifically inhibits gene expression in mammalian cell lines (Elbashir, S. M., et al.: (2001) Nature 411, 494-498). Particularly preferable siRNA of the present invention includes si-mimitin-1 (siRNA corresponding to 72-90 of mimitin) and si-mimitin-2 (siRNA corresponding to 330-348 of mimitin) prepared as described in Example 5.

Accordingly, the present invention encompasses a molecule inhibiting the cell proliferation activity of the protein of the present invention, which is a siRNA molecule containing a part of SEQ ID NO: 2 or SEQ ID NO: 4, and a complementary chain thereof. The siRNA of the present invention is 18-25 mer, preferably 21 mer, in length. A siRNA molecule relating to the base sequence shown in SEQ ID NO: 2 is preferable.

Such siRNA can be introduced into a cell by, for example, the method described in Elbashir, S.M., et al.: (2001) Nature 411, 494-498. Alternatively, a plasmid, a viral vector and the like designed to synthesize siRNA in a cell can be utilized (Wall NR, Shi Y., Small RNA: can RNA interference be exploited for therapy? (2003) Lancet, 362(9393):1401-1403.; Tuschl T, and Borkhardt A., Small interfering RNAs: a revolutionary tool for the analysis of gene function and gene therapy. (2002) Mol Interv. 2(3):158-67; Dave RS, and Pomerantz RJ, RNA interference: on the road to an alternate therapeutic strategy!, (2003) Rev Med Virol. 13(6):373-385; Sioud M., Therapeutic siRNAs. (2004) Trends Pharmacol Sci.25(1):22-28).

For a brief explanation, 24 hr before siRNA introduction, cells in the exponential growth phase are treated with trypsin, and a suitable amount of siRNA is introduced into the cells by a lipofection method including an oligofectamine method, a calcium phosphate method, an electroporation method and the like. The cells are cultured in a serum free medium for 4-20 hr or longer to introduce siRNA. The siRNA can be expressed in the cell using a siRNA expression vector such as pSilencer^{™} adeno 1.0-CMV (Ambion, Cat# 5796) and the like. At that time, the expression vector can be introduced into the cell by a lipofection method, a calcium phosphate method, an electroporation method and the like.

The inhibitory molecule inhibits the activity of mimitin, i.e., cancer cell proliferation activity. Accordingly, as another embodiment, the present invention relates to a pharmaceutical composition containing the inhibitory molecule of the present invention as an active ingredient, preferably a pharmaceutical composition for the prophylaxis or treatment of cancer, more preferably a pharmaceutical composition for the prophylaxis or treatment of esophageal cancer.

The inhibitory molecule utilizes a chemically synthesized siRNA molecule, or a plasmid, a viral vector and the like designed to synthesize siRNA in a cell ( Wall NR, Shi Y., Small RNA: can RNA interference be exploited for therapy? (2003) Lancet, 362(9393):1401-1403.; Tuschl T, and Borkhardt A., Small interfering RNAs: a revolutionary tool for the analysis of gene function and gene therapy. (2002) Mol Interv. 2(3):158-67; Dave RS, and Pomerantz RJ, RNA interference: on the road to an alternate therapeutic strategy!, (2003) Rev Med Virol. 13(6):373-385; Sioud M., Therapeutic siRNAs. (2004) Trends Pharmacol Sci.25(1):22-28).

The pharmaceutical composition of the present invention may contain a well-known excipient. As the administration method of the pharmaceutical composition, intradermal administration, subcutaneous administration and intravenous injection can be mentioned, and a dosage form suitable for each administration method is employed. While the content of the active ingredient of the present invention in a preparation can be appropriately adjusted according to, for example, the disease state to be treated, age, body weight of individual patient and the like, it is generally 0.001 - 100 wt%, which is administered once a day to once in several days.

As another embodiment, the present invention relates to a method comprising a step of screening for and evaluating a substance acting as an inhibitory substance of the biological activity induced by the binding of the polynucleotide molecule of the present invention and a c-Myc protein, by providing the relationship between a polynucleotide molecule of the present invention, particularly the polynucleotide molecule encoding Mimitin protein, and a c-Myc protein. That is, the present invention relates to a method of screening for a substance inhibiting the cell proliferation activity of the polynucleotide molecule of the present invention, which is a method comprising a step of adding a sample containing a candidate inhibitory substance to a reaction system containing the polynucleotide molecule and a c-Myc protein, and measuring the activity of the candidate substance inhibiting the binding of the polynucleotide molecule and the c-Myc protein.

The candidate substance of the inhibitory substance may be any known substance or novel substance including the aforementioned inhibitory molecule of the present invention and, for example, nucleic acid, carbohydrates, lipid, protein, peptide, organic low-molecular-weight compound, compound library prepared by a combinatorial chemistry technique, random peptide library prepared by solid phase synthesis or a phage display technique, a natural component derived from or microorganism, animal and plant, marine organism etc., and the like can be used. The activity to inhibit the binding of the aforementioned polynucleotide molecule and a c-Myc protein can be determined by a known method and the method is not particularly limited. For example, a method using the transcription activity of the c-Myc protein as an index can be mentioned. The transcription activity can be determined, for example, by introducing a reporter plasmid containing the polynucleotide molecule of the present invention (preferably DNA containing promoter region) and a reporter gene such as luciferase, GFP (green fluorescence protein), β-galactosidase, CAT (chloramphenicol acetyltransferase) and the like into a cell expressing Myc, contacting the aforementioned cell with a sample containing the aforementioned candidate substance, and determining the reporter activity by a conventional method. The obtained activity is compared with the activity of a cell free of a contact with the candidate substance, and a candidate substance showing lower activity can be selected as an inhibitory substance.

The thus-obtained inhibitory substance is considered to be usable for the prophylaxis or treatment of cancer, particularly esophageal cancer. Here, the cancer is not particularly limited as long as it consists of a cell or tissue that expresses both c-Myc and Mimitin and, for example, bladder cancer, breast cancer, colon cancer, gastric cancer, esophageal cancer, liver cancer, melanoma, myeloma, neuroblastoma, glioblastoma, ovarian cancer, prostate cancer, lung cancer and the like can be mentioned. The invention is effective for esophageal cancer, glioblastoma and breast cancer, and most effective for esophageal cancer.

As a further embodiment, the present invention relates to diagnosis of cancer, particularly esophageal cancer, specifically a method of detecting the protein of the present invention, a variant thereof or a fragment thereof in patients, which comprises taking a cancer tissue from a patient suspected of having a cancer, labeling the obtained section with an antibody against the protein of the present invention, Mimitin, or a variant thereof, and judging the level of labeling.

The cancer tissue taken from the patient suspected of having a cancer is, for example, fixed with 10% formalin, paraffin-embedded, and immunostained by a streptoavidin-biotin complex immunoperoxidase technique and the like.

Then, immunohistochemical staining is evaluated by a microscopic observation according to a conventional method.

Moreover, the present invention relates to a method for the prophylaxis or treatment of cancer, which comprises administering the pharmaceutical composition of the present invention to an animal.

As the animal to be the subject of administration, human and animals other than human, and as the animals other than human, mouse, rat, rabbit, guinea pig, hamster, swine, bovine, horse, sheep, goat, dog, cat, bird, monkey and the like can be mentioned.

While the dose and administration frequency can be appropriately adjusted depending on the disease state of the treatment subject, age and body weight of individual patient, and the like, it is generally 0.001 mg - 1000 mg, per day and the administration frequency is generally once a day to once in several days.

As the administration method, intradermal administration, subcutaneous administration and intravenous injection can be employed.

### Examples

The present invention is explained in more detail in the following by referring to Examples, which are not to be construed as limitative in any meaning.

### Example 1

### Cloning of c-Myc target gene, mimitin

A myc target gene, mimitin, was cloned by a method similar to the method of 5'-RACE and RT-PCR protocol as described in reference 1 (Tsuneoka M., Koda Y., Soejima M., Teye K., Kimura H. A novel Myc target gene, mina53, that is involved in cell proliferation. J. Biol. Chem. 277 (38): 35450-35459, 2002).

### (1) Establishment of cell line having conditionally activated c-Myc activity

To conditionally induce c-Myc activity, the estrogen-inducible Myc system was used (Eilers, M., et al.: (1989) Nature 340, 66-68).

Human glioblastoma cell line T98G cells (human, Caucasian, glioblastoma, ECACC 92090213, ATCC CRL 1690) were cultured in Eagle's medium supplemented with non-essential amino acids and 10% fetal calf serum (FCS). pc-mycer/CAGGS (Tsuneoka M., Koda Y., Soejima M., Teye K., Kimura H. A novel Myc target gene, mina53, that is involved in cell proliferation. J. Biol. Chem. 277 (38): 35450-35459, 2002)(20 µg) encoding a c-MycER chimeric protein and pActHyg (Tsuneoka M., Nakano F., Ohgusu H., and Mekada E., c-myc activates RCC1 gene expression through E-box elements, Oncogene, 14: 2301-2311, 1997)(0.4 µg) encoding a hygromycin resistance gene were transfected into T98G cells (Chen, C.A., et al.: (1988) Biotechniques 6, 632-638) by a calcium phosphate technique. The obtained cells were cultured in a medium containing 200 µg/ml of hygromycin for 2 weeks. Each clone was isolated, expression of c-MycER protein was detected by the Western blot analysis method using an anti-c-Myc antibody, and T98G cell that expressed c-mycer (hereinafter to be referred to as T98Gmycer-2 cells) was established.

A specific signal for ODC (Pena A, Reddy CD, Wu S, et al. Regulation of human ornithine decarboxylase expression by the c-Myc.Max protein complex. J Biol Chem 1993; 268: 27277-85), which is an already-reported Myc target gene, and a specific signal for nucleolin (Greasley PJ, Bonnard C, Amati B. Myc induces the nucleolin and BN51 genes: possible implications in ribosome biogenesis. Nucleic Acids Res. 2000;28:446-53) increased 2.6-fold and 1.6-fold, respectively, by a differential display analysis using a DNA chip (cDNA microarray) to be mentioned in the next item, due to the activity of c-MycER in T98Gmycer-2 cells. The results reveal that the prepared T98Gmycer-2 cells can detect a Myc target gene.

### (2) Differential display using DNA chip (cDNA microarray)

Total RNA was isolated from each of untreated T98Gmycer-2 cells and T98Gmycer-2 cells treated with 4-hydroxytamoxifen (OHT) for 20 hr. Poly(A+)RNA was recovered, and applied to differential display using a commercially available DNA chip (manufactured by Incyte). About 9 thousand kinds of cDNAs containing an expression sequence tag (EST) clone were plated on a chip (UniGEM Human V Ver. 2), and a gene showing an enhanced expression due to the c-MycER action was screened for. The signal for the EST clone AI803230 was stimulated 2.2-fold with c-Myc activity. This stimulation rate was similar to those of the Myc target genes, ODC, and nucleolin, measured as shown above.

### (3) 5'-RACE (Rapid Amplification of 5'cDNA Ends) and 3'-RACE analysis

cDNA encoding the 5' upstream of EST clone AI803230 was isolated using the 5'-RACE protocol from a library of human erythroid leukemia cells. cDNA encoding the 3' downstream of EST clone AI803230 was isolated using the 3'-RACE protocol from a library of human erythroid leukemia cells.

Polymerase Chain Reaction (PCR) amplification was performed using 50 µl of Ex Taq buffer containing 10 µmoles of each primer, Ex Taq DNA polymerase (1.2 U, manufactured by Takara Shuzo) and 200 µM of dNTP. For 5'-RACE, from poly(A)+RNA (1 µg) of HEL cells (Koda, Y. et al.: (1997) J. Biol. Chem. 272, 7501-7505), a reverse transcription, double-stranded cDNA synthesis and adaptor-ligation were performed using Marathon cDNA amplification kit (manufactured by Clontech) (Koda, Y. et al.: (1997) J. Biol. Chem. 272, 7501-7505). The first PCR was performed using the primers, mimitin-RACE-1 (5'-GCTGCTGGGAGCCACTGAGGGTTCT-3': SEQ ID NO: 5) and the AP1 primer (manufactured by Clontech, 5'-CCATCCTAATACGACTGACTATAGGGC-3': SEQ ID NO: 6). As the temperature profile, a treatment of 25 cycles was performed, which contained, after initial heat denaturation at 94°C for 1 min, a heat denaturation step at 96°C for 15 sec, and an annealing step and elongation step at 68°C for 3 min as 1 cycle. A solution (1 µl) obtained by diluting (1000-fold) the above-mentioned first RACE-PCR product was used as a template for nested RACE-PCR. For the nested RACE-PCR, mimitin-RACE-2 (5'-GCATGGCCTTTAATTTGAGTTTGAAC-3': SEQ ID NO: 7) and AP2 primer (manufactured by Clontech, 5'-ACTCACTATAGGGCTCGAGCGGC-3': SEQ ID NO: 8) were used. As the temperature profile, a treatment of 25 cycles was performed, which contained, after initial heat denaturation at 94°C for 1 min, a heat denaturation step at 96°C for 15 sec, and an annealing step and elongation step at 68°C for 4 min as 1 cycle. The amplified 0.6 kb DNA fragment was cloned into a pGEMT vector and sequenced. A primer for 3'-RACE was designed based on the sequence information.

For 3'-RACE, from poly (A) +RNA (1 µg) of HEL cells (Koda, Y. et al.: (1997) J. Biol. Chem. 272, 7501-7505), a reverse transcription, double-stranded cDNA synthesis and adaptor-ligation were performed using Marathon cDNA amplification kit (manufactured by Clontech) (Koda, Y. et al.: (1997) J. Biol. Chem. 272, 7501-7505). For the first PCR, mimitin-3' RACE-1 (5'-GCGGCTGGAGCATTACCCCTACTGC-3': SEQ ID NO: 9) and AP1 primer were used as primers. As the temperature profile, a treatment of 25 cycles was performed, which contained, after initial heat denaturation at 94°C for 1 min, a heat denaturation step at 96°C for 15 sec, and an annealing step and elongation step at 68°C for 3 min as 1 cycle. A solution (1 µl) obtained by diluting (1000-fold) the above-mentioned first RACE-PCR product was used as a template for nested RACE-PCR. For nested RACE-PCR, mimitin-3'RACE-2 (5'-AAACTGGGTGGACGGCATGGGTTGG-3': SEQ ID NO: 10) and AP2 primer were used. As the temperature profile, a treatment of 25 cycles was performed, which contained, after initial heat denaturation at 94°C for 1 min, a heat denaturation step at 96°C for 15 sec, and an annealing step and elongation step at 68°C for 4 min as 1 cycle. The amplified 0.6-kb DNA fragment was cloned into a pGEMT vector, and the obtained vector was called pT/mimitin.

### (4) RT-PCR (Reverse Transcriptase-PCR)

The entire sequence of the mRNA molecule was predicted based on the nucleotide sequence from the RACE experiment. As a result, it was found that all nucleotides encoding a protein were present in the aforementioned pT/mimitin. The determined cDNA sequence of human is shown in SEQ ID NO: 1. It was found that mimitin DNA encodes the 169-amino acid protein having a predicted molecular mass of 19856.33 Da.

Mimitin-RT-F (5'-CCGGAATTCTGCCATGGGTTGGTCTCAGGATTTGTTCC-3': SEQ ID NO 11, adding an EcoRI site at 5' end), and mimitin-RT-R (5'-GGAAGATCTGAATTCATTGATTGTGGCTCTTGCCATCTCG-3': SEQ ID NO 12, adding an EcoRI site at 3' end), as primers for PCR amplification. As the temperature profile, a treatment of 35 cycles was performed, which contained a heat denaturation step at 98°C for 15 sec, an annealing step at 65°C for 1 min and an elongation step at 72°C for 2.5 min as 1 cycle.

Then, amplified cDNA was treated with EcoRI and cloned into a pCAGGS vector previously cleaved with EcoRI and dephosphorylated at the terminal to give pCAGGS/mimitin.

As for mouse, a single-stranded cDNA of the cell of the stomach of mouse was synthesized from total RNA (1 µg) using a commercially available Superscript single-stranded cDNA synthesis kit (Invitrogen). The obtained single stranded cDNA (1 µl, total amount 20 µl) was used as a template for PCR. A mouse cDNA fragment having a sequence homologous to the human sequence was searched from the GenBank Data base and amplified using a polynucleotide encoding mouse mimitin as a primer for RT-PCR, and mouse mimitin RT-F (5'-CCGGAATTCAGGCAGGCATGAGCTGGTGGTCCGGTGTGTGGCGC-3': SEQ ID NO: 13) and mouse mimitin RT-R (5'-GGTCGAATTCATTGACTTTGTCTTTTGCCATCTTC-3': SEQ ID NO: 14). As the temperature profile, a treatment of 35 cycles was performed, which contained a heat denaturation step at 98°C for 15 sec, an annealing step at 65°C for 1 min and an elongation step at 72°C for 2.5 min as 1 cycle.

Then, the amplified cDNA was cloned into a pGEM-T vector (manufactured by Promega), and the sequence thereof was determined. The cDNA sequence of the obtained mouse is shown in SEQ ID NO: 3. The mouse mimitin codes for a protein consisting of 168 amino acids and having a predicted molecular mass of 19627.85.

### Example 2

### Preparation of Mimitin protein

Using, as primers for PCR for amplification, mimitin-RT-F (SEQ ID NO: 11, added with EcoRI site to 5'-end) and mimitin-RT-R (SEQ ID NO: 12, added with EcoRI site to 3'-end), 0.5 kb length cDNA was amplified according to PCR protocol and using pT/mimitin as a template, a 0.5 kb EcoRI fragment was inserted into E. coli expression vector pET28 (Novagen) (cleaved with EcoRI and dephosphorylated with E. coli alkali phosphatase) to give pET/mimitin (encoding Mimitin protein with His tag). The above-mentioned 0.5 kb EcoRI fragment was inserted into pGEX-3X (Pharmacia)(digested with EcoRI and dephosphorylated with E. coli alkaliphosphatase) to give pGEX/mimitin (encoding GST fusion Mimitin protein). Mimitin protein was produced by Escherichia coli by a conventional method and using the above-mentioned plasmid, and Mimitin protein having a His tag was purified using a nickel affinity column, and GST fusion Mimitin protein was purified using a glutathione affinity column.

### Example 3

### Expression of mimitin

Using an antigen prepared in Example 2, an anti-Mimitin rabbit antibody was prepared by a conventional method. Using the antibody, human glioblastoma cell line T98G cells were basically stained by an indirect immunofluorescence basically according to a method already described (Tsuneoka M., and Mekada E., 1992, J. Biol. Chem., 267: 9107-9111), whereby the cytoplasm was stained.

### (1) Induced expression of Mimitin protein

T98G cells and T98G mycer cells were treated with OHT (4-hydroxytamoxifen) according to a known publication (J. Biol. Chem., 277: 35450-35459 (2002)) and further cultured for 40 hr.

The aforementioned cells were then recovered, and expression of Mimitin protein was examined by Western blotting and using an anti-Mimitin rabbit antibody (Fig. 1A, left panel) .

Then, a vector expressing Mimitin and a vector expressing Mimitin labeled with c-myc were each introduced into T98G cells, and the expression of Mimitin protein was examined in the same manner as above (Fig. 1A, right panel).

As a result, it was clarified that Mimitin protein was detected as a 20kd band predicted from the amino acid sequence, the expression of Mimitin protein increased by activation of c-MycER with OHT, its expression induction did not accompany stimulation of cell proliferation, when exogeneous Mimitin was expressed, a 20 kd band was similarly detected, and when Mimitin labeled with c-myc was expressed, mobility became slow.

### (2) Intracellular localization of Mimitin protein

HeLa cells grown on glass coverslips in 6-well plate were fixed in 4% paraformaldehyde for 30 min at room temperature, and left in PBS containing 1% Triton X-100. An anti-Mimitin rabbit antibody was added thereto and the cells were incubated at 37°C for 120 min. After washing 3 times with a 0.1% skim milk in PBS suspension, Alexa488-conjugated anti-rabbit IgG was added, and the cells were reacted at 37°C for 120 min and washed 3 times with 0.1% skim milk in PBS suspension. Finally, the cells were embedded in Immunon (manufactured by Terumo Shandon), observed with a fluorescence microscope and photographed. In the same manner, the cells were stained by an indirect immunofluorescence using an antibody against Tom20 localized in the external membrane of mitochondria, observed with a fluorescence microscope, and photographed. The obtained fluorescence stained images and an overlapped image (merge) of the two kinds of stained images are shown in Fig. 1B. From Fig. 1B, it was clarified that the Mimitin protein showed a stain image similar to that of Tom20, and Mimitin was a protein localized in mitochondria.

Human glioblastoma cell line T98G cells were fractionated into enriched mitochondrial and cytosolic fractions using a mitochondria/cytosol fractionation kit (BioVision Research Products, Mountain View, CA, catalog number K256-100), and extracts derived from the respective fractions were subjected to Western blotting. The antibodies used were the above-mentioned anti-Mimitin rabbit antibody, an antibody against mitochondria protein Tom22 (J. Biol. Chem. 275, 31996-32002), and an anti- MAPK/ERK-1 antibody (K-23, Santa Cruz Biotechnology). The results are shown in Fig. 1C. From Fig. 1C, it was clarified that Mimitin is localized in a mitochondria fraction, like Tom22.

### (3) Analysis of expression of mimitin

The state of Mimitin expression was examined by Northern blot.

First, each DNA probe of c-myc, mimitin and GAPDH was prepared.

### Preparation of c-myc probe:

A 1.6 kb HindIII-XbaI fragment of pc-myc/CDM8 (Tsuneoka M., Nakano F., Ohgusu H., and Mekada E., c-myc activates RCC1 gene expression through E-box elements, Oncogene, 14: 2301-2311, 1997) containing human c-myc cDNA was prepared (Tsuneoka, M., et al.: (1997) Oncogene 14, 2301-2311).

### Preparation of mimitin probe:

The 0.5 kbp fragment prepared in Example 2 was used.

### Preparation of GAPDH probe:

A 1.8 kb fragment obtained by amplification according to the RT-PCR technique, using 5'-TGAAGGTCGGAGTCAACGGATTTGGT-3' (SEQ ID NO: 15) and 5'-CATGTGGGCCATGAGGTCCACCAC-3' (SEQ ID NO: 16), from the total RNA isolated from HL60 cells cultured in 10 nM of TPA for 24 hr. The sequence of GAPDH cDNA was confirmed by the direct sequence method.

Total RNA was isolated from human glioblastoma cell line T98G cells (human, Caucasian, glioblastoma, ECACC 92090213, ATCC CRL 1690) and human promyelocytic leukemia cells HL60 (ECACC 85011431, ATCC CCL 240) using a commercially available RNA preparation liquid set (manufactured by Nacalai Tesque) and according to the acid guanidium-thiocyanate-phenolchloroform extraction method. The extracted RNA was electrophoresed using a formaldehyde-containing agarose gel, transferred to Hybond N (manufactured by Amersham Biosciences), and detected with each ³²P-labeled cDNA probe prepared in advance. Each probe was labeled with [α-³²P]dCTP using a commercially available Multiprime labeling kit (manufactured by Amersham Biosciences). The results were quantified by a BAS200 Image analyzer (manufactured by Fujifilm Corp.).

Human glioblastoma cell line T98G cells were subjected to serum starvation by culturing with Eagle's medium containing non-essential amino acids and a 0.25% fetal calf serum (FCS) for 2 days. Then, the cells were subjected to serum stimulation by adding fetal calf serum to the final concentration of 10%. As a result, increased c-myc expression and then increased mimitin expression were observed (Fig. 2A). TPA (tetradecanoyl phorbol acetate) was added to a culture medium of human promyelocytic leukemia cells HL60 to the final concentration of 10 nM. As a result, decreased myc expression, and then decreased mimitin expression were observed (Fig. 2B). These indicate that the c-myc expression and the mimitin expression are correlated.

The T98G mycer cells expressing MycER, which were prepared in Example 1(1), were treated with OHT. As a result, mimitin expression increased and the increase was also observed in the presence of a protein synthesis inhibitor (CHX, cycloheximide) (Fig. 2C). Therefrom it was found that c-Myc did not require new protein synthesis and directly enhanced the mimitin expression.

### (4) Analysis of a mechanism for enhancing mimitin expression by c-Myc

A genomic DNA fragment of the human mimitin gene, which extends from the promoter region to intron 1, was amplified by PCR using 5'-GTGGAGGGCAGTTATTCTTTGGAG-3' (SEQ ID NO: 17) and 5'-GGGCTCTCACTTTTCCTTCCTTTA-3' (SEQ ID NO: 18), as a primer and using a DNA derived from human peripheral blood as a template, and cloned into a pGEM-T vector (Promega). The DNA fragment was amplified from the plasmid, as a template, with 5'-GTGGAGGGCAGTTATTCTTTGGAG-3' (SEQ ID NO: 19, adding a MluI site) and 5'-GGGGCTCCCATGGTTCCTTCCTTTACTGACCTC-3' (SEQ ID NO: 20, adding a NcoI site). The obtained 0.9-kb amplified fragment was cleaved with MluI and NcoI, and inserted into the 4.8-kb fragment of a pGL3-basic (Promega), which was cleaved with MluI and NcoI to produce pmimitin(W)luci. By using Gene Editor in vitro Site-Directed Mutagenesis System (Promega), mutations were introduced to E-box site of pmimitin(W)luci (the CACGTG element), to produce pmimitin(m)luci, in which CACGTG was mutated to CACCCG.

T98Gmycer-2 cells were cultured in medium supplemented with 0.25% fetal calf serum. 3x10⁴ cells were plated in a dish (12 well plate; well diameter: 22 mm) and cultured for 20-24 hr. Reporter plasmid (1 µg) and 20 ng of pRL-CMV (Promega, expresses Renilla reniformis luciferase with CMV promoter) as an internal marker were introduced into the cells using an FuGENE6 reagent (manufactured by Roche Diagnostics). After 10 hr from the introduction of the reporter gene, OHT was added to the final concentration of 0.2 µM to activate the MycER chimeric protein and the cells were further cultured for 9-12 hr. Then, the cells were collected, and the activity of firefly luciferase and Renilla reniformis luciferase was quantified using a dual-luciferase reporter assay system (manufactured by Promega). Gene introduction levels of the samples were standardized using the Renilla reniformis luciferase activity value, and the firefly luciferase activity of the cells containing activated MycER was shown in a ratio to that of the cells free of activated MycER. To standardize the effect of OHT free from activation of MycER, the value of the T98Gmycer-2 cells was divided by the value of T98G parent cells treated in the same manner as in the above-mentioned T98Gmycer-2 cells, and used to show the reactivity of the reporter gene relative to the activity of the MycER chimeric protein. The obtained results are shown in Fig. 3A. In the Figure, pBasic shows cells into which a vector alone was introduced, wild shows cells into which a CACGTG element was introduced, and mutant shows cells into which a mutated element was introduced. The value is an average of 4 experiments.

The promoter region of the mimitin gene was excised as mentioned above, ligated with luciferase cDNA, and a reporter gene to determine the mimitin promoter activity was constructed. The expression of luciferase from the reporter gene increased as a result of activation of c-Myc (MycER). c-Myc is known to bind to a sequence, CACGTG, and enhance transcription of the gene. When the mutation was introduced into the sequence, CACGTG, in the promoter derived from the Mimitin gene, enhanced expression by c-Myc (MycER) was not seen (Fig. 3A). This indicates that c-Myc enhances mimitin expression via the CACGTG sequence.

### (5) Chromatin immunoprecipitation experiment

To examine whether the c-Myc protein is directly bound with the intracellular mimitin genome gene, a chromatin immunoprecipitation experiment (Chip) was performed using leukemia cells HL60 treated with TPA.

The chromatin immunoprecipitation experiment was performed using an antibody against c-Myc and a control antibody, and a control experiment was also performed without using an antibody. In this experiment, 2 kinds of antibodies against c-Myc were used. One was antibody-1 (Santa Cruz Biotechnology, Santa Cruz, CA Cat# N262) reactive with a half of the N-terminal of c-Myc, and the other was antibody-2 (Tsuneoka et al., 1997, Oncogene, 14: 2301-2311) mainly bound with a half of the C-terminal of c-Myc. The assay was basically performed according to an already published method (Boyd, K.E., et al.: (1997) Mol. Cell. Biol 17, 2529-2537). The immunocomplex was collected by the addition of 20 µl of protein A beads (50% v/v) treated to prevent nonspecific binding. As described in the above-mentioned publication, the beads were washed, a DNA fragment was eluted, and the eluted solution was extracted with phenol/chloroform and precipitated with ethanol. Immunoprecipitated DNA fragments were detected by PCR, using the primers 5'-GTGGAGGGCAGTTATTCTTTGGAG-3' (SEQ ID NO: 17) and 5'-GGGCTCTCACTTTTCCTTCCTTTA-3' (SEQ ID NO: 18), which amplified a 0.9-kb fragment containing E-box located in exon 1 of the human mimitin gene. As other primers, 5'-TTACAGGTAAGCCCTCCAATGACC-3' (SEQ ID NO: 21) and 5'-GCAAAGCTACCATTTAGGAACCC-3' (SEQ ID NO: 22) were used, and the genomic sequence in the E-box located in the region free of a detectable gene in the chromosome 22 (Bouchard, C., et al.: (2001) Genes & Dev 15, 2042-2047) was amplified. The E-box is present in a chromosome having no detectable gene (Bouchard, C., et al.: (2001) Genes & Dev 15, 2042-2047).

As mentioned above, mimitin genome fragments were recovered by two kinds of anti-c-Myc antibodies, but otherwise by the control antibody (Fig. 3B). When Myc expression was suppressed in advance by a TPA treatment, a mimitin genome fragment was not recovered by an anti-c-Myc antibody. A genome fragment free of a gene but containing the CACGTG sequence was not recovered in any case by an anti-c-Myc antibody. These suggest that Myc is directly bound with the mimitin genome.

From the above, it has been established that c-Myc is directly bound with the mimitin genome gene and increases its expression.

### Example 4

### Analysis of expression of mimitin in esophageal cancer cells

The esophageal cancer tissue obtained in Kurume University was examined for the presence of Mimitin protein by detection using an anti-Mimitin antibody. The detection was performed according to a method similar to the one described in reference literature (Teye K., Tsuneoka M, Arima N., Koda Y., Nakamura Y., Ueta Y, Shirouzu K., and Kimura H. Increased Expression of a Myc Target Gene mina53 in Human Colon Cancer. The American Journal of Pathology 164(1) : 205-216., 2004).

The GST fusion Mimitin protein prepared according to Example 2 and purified using a glutathione affinity column was further applied to SDS-PAGE for preparation, thereby completely separating the protein from other proteins, and used as an antigen for production of an antibody against Mimitin protein. Using this antigen, an anti-Mimitin serum was prepared by a conventional method. The anti-Mimitin serum was purified by a His-tagged Mimitin protein purified using a nickel affinity column and used as an anti-Mimitin antibody. Using this antibody, protein Mimitin in an esophageal cancer tissue was detected, and cancer region was identified using the HE staining method. A 10% formalin-fixed paraffin-embedded esophageal cancer tissue was deparaffinized, and immunnostained by the streptoavidin-biotin complex immunoperoxidase method (Fujitani, N., et al.: J Histochem Cytochem 2000, 48:1649-1656). To recover antigenicity, a slide mounting a section was autoclaved for 20 min in a 10 mM sodium citrate buffer (pH 6.0). After a pretreatment with a solution of 3% hydrogen peroxide in PBS and then with a PBS solution containing 1% skim milk and 5% rabbit serum, the slide was reacted with a 1% skim milk solution containing a final concentration of 3.5 µg/ml of an anti-Mimitin antibody or an anti-Ki-67 antibody in a wet chamber at 4°C for one whole day and night. After washing 3 times with a solution of 0.05 % Tween in PBS, the section was incubated with a biotinated anti-rabbit IgG goat antibody or biotinated anti-mouse IgG rabbit antibody, and successively with an HRP-streptoavidin conjugate. Then, using 3,3-diaminobenzidine and hydrogen peroxide, the slide was developed for 2 min and washed with water to quench the reaction. After counterstaining with hematoxylin, the slide was dehydrated, covered with a glass plate, and observed with a microscope.

Each section was scored from 0 to 4 by visual observation. Score of the section showing the highest staining intensity was 4, and score of the section free of staining was 0. To estimate the percentage of stained cells, the numbers of positive and negative cells in a field of the vision were counted and expressed as the percentage of cells stained. For a staining index, the staining intensity was multiplied by an average percentage (%) of the stained cells and the obtained value was used. The section shown in Fig. 4 contained moderately differentiated esophageal carcinoma cells (Fig. 4A). As shown in Fig. 4B, the intratumor area was remarkably stained with an anti-Mimitin antibody. The staining was mainly found in the cytoplasm (Fig. 4B). When the section was treated without using the antibody (Fig. 4C), staining did not occur. The stained cells highly overlapped with the region stained with an anti-c-Myc antibody (Fig. 4D). It does not contradict with the fact that mimitin is a Myc target gene. When stained with an anti-Ki-67 antibody widely used for detecting cells under growth, the intratumor area was remarkably stained, and the stained area overlapped with the area stained with an anti-Mimitin antibody (Fig. 4E). From these results, it is suggested that Mimitin is associated with cell proliferation in esophageal carcinoma cells.

Then, tumor sections from 35 esophageal cancer patients were stained and staining intensity, proportion (%) of the stained cells and staining index of each of them were determined (Table 1). In addition, expression in the esophagus normal epithelium was determined in 23 cases in the same manner as in the tumor region, and the average staining index was calculated to be 0.805. A tumor showing a value higher than the staining index of Mimitin in normal epithelium was evaluated to be a Mimitin high expression tumor. As a result, high expression of Mimitin was observed in 80% (28 cases out of 35 cases). Therefrom it was clarified that Mimitin expression is characteristic to esophageal cancer.

The Mimitin staining index was compared with biological characteristics of tumor (level of differentiation, progress, growth level (Fig. 4E)). As the anti-Ki-67 antibody, a mouse monoclonal antibody (MIB-1) (DAKO, Glostrup, Denmark) was used. As regards Ki-67, the staining intensity, proportion (%) of the stained cells and staining index of tumor sections from 35 esophageal cancer patients were also determined in the same manner as in Mimitin. As regards c-Myc, the staining intensity, proportion (%) of the stained cells and staining index of tumor sections from 35 esophageal cancer patients were also determined in the same manner as in Mimitin. The obtained results are shown in Table 1 and Table 2. In Table 2, r shows the correlation. A + and higher value thereof shows the presence of a more positive correlation. P shows a p value. When p is not more than 0.05, the correlation is considered to be significant.

**[Table 1] esophageal cancer patients and histological characteristics thereof**

| patient No. (sex, age) | level of progression of cancer | differentiation level # | Mimitin stain | | | Ki-67 stain | | | c-Myc stain | | |
|---|---|---|---|---|---|---|---|---|---|---|---|
| | | | intensity | % | stain level | intensity | % | stain level | intensity | % | stain level |
| 1 (M, 62) | I | G2 | 2 | 86 | 1.72 | 3 | 89 | 2.67 | 1 | 58 | 0.58 |
| 2(M, 72) | III | G1 | 1 | 56 | 0.56 | 3 | 83 | 2.49 | 2 | 82 | 1.64 |
| 3 (M, 77) | I | G3 | 2 | 71 | 1.42 | 1 | 12 | 0.12 | 2 | 79 | 1.58 |
| 4 (M, 56) | III | G1 | 3 | 93 | 2.79 | 3 | 84 | 2.52 | 2 | 90 | 1.8 |
| 5 (M, 59) | III | G1 | 2 | 82 | 1.64 | 3 | 64 | 1.92 | 2 | 85 | 1.7 |
| 6 (M, 60) | IIa | G1 | 2 | 91 | 1.82 | 3 | 63 | 1.89 | 2 | 87 | 1.74 |
| 7 (M, 67) | IV | G2 | 2 | 91 | 1.82 | 3 | 49 | 1.47 | 0.5 | 19 | 0.1 |
| 8(M,67) | III | G2 | 2 | 85 | 1.7 | 3 | 39 | 1.17 | 0 | 0 | 0 |
| 9 (M, 69) | III | G2 | 3 | 85 | 2.55 | 4 | 71 | 2.84 | 3 | 81 | 2.43 |
| 10 (M, 51) | I | G2 | 3 | 34 | 1.02 | 0 | 0 | 0 | 0.5 | 56 | 0.28 |
| 11 (M, 49) | III | G2 | 3 | 67 | 2.01 | 3 | 84 | 2.52 | 3 | 94 | 2.82 |
| 12 (M, 47) | I | G3 | 3 | 95 | 2.85 | 3 | 65 | 1.95 | 3 | 92 | 2.76 |
| 13 (M, 57) | IV | G1 | 3 | 92 | 2.76 | 4 | 60 | 2.4 | 0.5 | 55 | 0.28 |
| 14 (M, 69) | III | G2 | 3 | 95 | 2.85 | 3 | 71 | 2.13 | 3 | 90 | 2.7 |
| 15(F,71) | IIa | G2 | 2 | 95 | 1.9 | 1 | 26 | 0.26 | 3 | 90 | 2.7 |
| 16 (M, 64) | III | G1 | 2 | 63 | 1.26 | 3 | 42 | 1.26 | 2 | 97 | 1.94 |
| 17 (M, 59) | III | G2 | 1 | 30 | 0.3 | 0 | 0 | 0 | 3 | 98 | 2.94 |
| 18 (M, 46) | III | G2 | 2 | 60 | 1.2 | 3 | 51 | 1.53 | 3 | 75 | 2.25 |
| 19 (M, 70) | I | G2 | 2 | 82 | 1.64 | 4 | 40 | 1.6 | 3 | 98 | 2.94 |
| 20 (M, 68) | IV | G2 | 2 | 44 | 0.88 | 0 | 0 | 0 | 2 | 64 | 1.28 |
| 21 (F, 76) | I | G2 | 2 | 95 | 1.9 | 4 | 55 | 2.2 | 3 | 90 | 2.7 |
| 22 (M, 68) | IIa | G1 | 1 | 43 | 0.43 | 0.5 | 6 | 0.03 | 2 | 47 | 0.94 |
| 23 (M, 41) | IIa | G3 | 2 | 43 | 0.86 | 3 | 30 | 0.9 | 1 | 46 | 0.46 |
| 24 (M, 66) | III | G2 | 2 | 32 | 0.64 | 2 | 18 | 0.36 | 1 | 53 | 0.53 |
| 25 (M, 65) | III | G2 | 3 | 92 | 2.76 | 2 | 18 | 0.36 | 3 | 57 | 1.71 |
| 26 (M, 66) | IIa | G1 | 3 | 88 | 2.64 | 4 | 72 | 2.88 | 3 | 90 | 2.7 |
| 27 (F, 71) | III | G3 | 3 | 94 | 2.82 | 4 | 95 | 3.8 | 2 | 79 | 1.58 |
| 28 (M, 50) | IV | G1 | 2 | 63 | 1.26 | 2 | 49 | 0.98 | 2 | 91 | 1.82 |
| 29 (M, 61) | IV | G1 | 4 | 98 | 3.92 | 4 | 93 | 3.72 | 3 | 93 | 2.79 |
| 30 (M, 67) | IV | G3 | 2 | 98 | 1.96 | 2 | 48 | 0.96 | 0 | 0 | 0 |
| 31 (M, 66) | IIb | G3 | 1 | 72 | 0.72 | 0 | 0 | 0 | 0 | 0 | 0 |
| 32 (M, 57) | IV | G1 | 2 | 78 | 1.56 | 4 | 47 | 1.88 | 3 | 81 | 2.43 |
| 33 (M, 61) | III | G1 | 0 | 0 | 0 | 0.5 | 6 | 0.03 | 0 | 0 | 0 |
| 34 (M, 80) | IIa | G1 | 0 | 0 | 0 | 0 | 0 | 0 | 0 | 0 | 0 |
| 35 (M, 63) | III | G2 | 2 | 95 | 1.9 | 3 | 47 | 1.41 | 3 | 96 | 2.88 |

| | | | | | | | | | | | |
|---|---|---|---|---|---|---|---|---|---|---|---|
| #G1, high differentiation; G2, moderate differentiation; G3, low differentiation. | | | | | | | | | | | |

**[Table 2]**

| Biological event in esophageal squamous cell carcinoma cells (ESCC) and relationship with Mimitin | | |
|---|---|---|
| factor | r | P |
| histopathological grade | 0.070 | 0.6901 |
| progression level of cancer | 0.131 | 0.4545 |
| age of patient | -0.069 | 0.6973 |
| c-Myc stain index | 0.435 | 0.0084 |
| cell proliferation (stain index of Ki-67) | 0.728 | <0.0001 |

The staining pattern by the anti-Mimitin antibody was highly similar to that by the anti-Ki-67 antibody. Ki-67 was expressed only in the cells under proliferation, and an anti-Ki-67 antibody is widely used for searching the cells under proliferation in a tissue. The fact that the cells stained by the anti-Mimitin antibody were also stained by the anti-Ki-67 antibody means that the cells showing Mimitin expression are under growth. From the above, it has been clarified that the Mimitin expression is related to cell proliferation.

### Example 5

### Involvement of Mimitin in growth of various culture cells

To investigate the biological function of mimitin, the relationship between the reduction of expression of Mimitin by RNA interference and the cell proliferation, using colon carcinoma SW620, cervical carcinoma HeLa, ESCC TE-11, T98G, embryonic lung HEL, and hepatocellular carcinoma Kyn2 cells.

Mimitin-specific siRNA (si-mimitin-2 of Example 6 below) was introduced into cells by a method substantially the same as the method described in a literature (Elbashir, S.M., et al.: (2001) Nature 411, 494-498). At 24 hr before siRNA introduction, the cells in the exponential growth phase were treated with trypsin, and plated on a 12-well plate. siRNA (200 pmole per well) was introduced into the cells using a commercially available Oligofectamine (Life Technology). The cells were cultured in a serum-free medium for not less than 20 hr to introduce siRNA. When Mimitin-specific siRNA was introduced into the culture cells by this method, the mimitin expression decreased in all the examined cells (Fig. 5A).

The culture cells into which siRNA was introduced and the culture cell free of the introduction were cultured further, and the level of proliferation was examined by counting the cells. The results are shown in Fig. 5B. From Fig. 5B, reduced cell proliferation of TE-11, T98G, and HEL cells was observed, but SW620, HeLa, and Kyn2 cells were not reduced.

The levels of c-Myc in the aforementioned 6 kinds of cells were compared by Western blotting (Fig. 5C). As shown in Fig. 5C, cell lines SW620 and HeLa, which proliferated without being affected by mimitin knockdown, contained high levels of c-Myc. c-Myc expression levels were relatively low and of the same level in TE-11, T98G, and Kyn2 cells.

In this group, mimitin knockdown suppressed cell proliferation in TE-11 and T98G cells, but not in Kyn2 cells. The c-Myc expression level was very low in HEL cells, whose cell proliferation was weakly suppressed by mimitin knockdown. Using TE-11 cells that showed the highest suppressive effect, the analysis was performed further.

### Example 6

### Functional analysis of Mimitin using siRNA

For a direct observation of the involvement of Mimitin protein in the growth of esophageal cancer cells, an in vitro experiment was conducted using cultured esophageal tumor cells.

First, cultured esophageal carcinoma cells TE-11 (provided by Institute of Development, Aging and Cancer, Tohoku University, Cancer cell Repository Center, esophageal tumor, squamous cell carcinoma, TKG 0262: TE-11) were treated with c-myc siRNA (Cat# 4604; Ambion Austin, TX), and c-Myc and Mimitin protein were detected by Western blot analysis using an anti-c-Myc antibody and an anti-Mimitin antibody. As a result, it was found that decreased c-Myc expression led to decreased Mimitin protein expression (Fig. 6A). This indicates that Mimitin expression is also controlled by c-Myc in the cultured esophageal carcinoma cells.

Then, siRNA double stranded si-mimitin-1 and si-mimitin-2 specific to human mimitin consisting of 21 nucleotides were chemically synthesized by a conventional method:
Si-mimitin-1; a part corresponding to 72nd - 90th from the first nucleotide of mimitin cDNA initiation codon was synthesized with ribonucleotide, and two dTs (deoxyribonucleotide) were added to the 3' terminal thereof. Produced by annealing 5'-CACGGACCAAUUCGGGAACdTdT (SEQ ID NO: 23) and 5'-GUUCCCGAAUUGGUCCGUGdTdT (SEQ ID NO: 24) (wherein dT is deoxyribonucleotide and others are ribonucleotides); and
Si-mimitin-2; a part corresponding to 330th - 348th from the first nucleotide of mimitin cDNA initiation codon was synthesized with ribonucleotide, and two dTs (deoxyribonucleotide) were added to the 3' terminal thereof. Produced by annealing 5'-ACUCCUUAGUAAAGAGACCdTdT (SEQ ID NO: 25) and 5'-GGUCUCUUUACUAAGGAGUdTdT (SEQ ID NO: 26) (where dT is deoxyribonucleotide and others are ribonucleotides).

As a nonspecific control, a commercially available control siRNA (Ambion Cat# 4611) was used.

Then, they were introduced into the cells by a method substantially the same as the method described in a literature (Elbashir, S.M., et al.: (2001) Nature 411, 494-498). At 24 hr before siRNA introduction, cells in the exponential growth phase were treated with trypsin, and plated on a 12-well plate. For introduction of siRNA into the cells, siRNA (200 pmole per well) and a commercially available Oligofectamine (LifeTechnology) were used. For introduction of siRNA, the cells were cultured in a serum-free medium for not less than 20 hr.

When si-mimitin-1 and si-mimitin-2 were applied to specifically prevent expression of mimitin, expression of Mimitin protein specifically decreased in esophageal tumor cells TE-11. In contrast, the expression of Mimitin protein did not decrease by control siRNA. It was observed that decreased expression of Mimitin protein caused marked inhibition of cell proliferation. These results show the importance of mimitin in the growth of esophageal cancer cells.

Mimitin expression could be suppressed by both si-mimitin-1 siRNA and si-mimitin-2 siRNA. Si-mimitin-2 siRNA showed stronger inhibition of expression than by si-mimitin-1 siRNA (Fig. 6A). Observation of cell proliferation at that time revealed that inhibition of mimitin expression resulted in strong inhibition of cell proliferation (Fig. 6B). The level of growth inhibition was higher in si-mimitin-2 siRNA than in si-mimitin-1 siRNA, which result correlating with the level of inhibition of expression. That is, a stronger inhibition of mimitin expression resulted in a stronger inhibition of cell proliferation. Thus, it is clear that the inhibition of mimitin expression is correlated to the inhibition of cell proliferation.

In the cells, inhibition of cell proliferation was observed when c-Myc expression was inhibited by c-myc siRNA. This indicates that c-myc expression is necessary for the cell growth. c-Myc has a function to increase mimitin expression. Since mimitin is necessary for the growth of the cells, mimitin gene is considered to be involved in the cell proliferation promoting action by c-Myc.

From the above, it has been clarified that mimitin is a novel Myc target gene, which is necessary for the proliferation of esophageal cancer cells. Therefrom mimitin is considered to have a potential to be applicable to the diagnosis or treatment of esophageal cancer.

Sequence listing free text
SEQ ID NO: 23; n is deoxythymine (located at 20-21).
SEQ ID NO: 24; n is deoxythymine (located at 20-21).
SEQ ID NO: 25; n is deoxythymine (located at 20-21).
SEQ ID NO: 26; n is deoxythymine (located at 20-21).

### Industrial Applicability

The mimitin gene is considered to be applicable to the diagnosis or treatment of cancer such as esophageal cancer and the like. An inhibitor (inhibitory substance) that prevents the action of the mimitin gene has a potential to be a new anti-cancer agent.

While some of the embodiments of the present invention have been described in detail in the above, it is, however, possible for those of ordinary skill in the art to make various modifications and changes to the particular embodiments shown without substantially departing from the teaching and advantages of the present invention. Such modifications and changes are encompassed in the spirit and scope of the present invention as set forth in the appended claims.

This application is based on a patent application No. 2004-147964 (filing date: May 18, 2004) filed in Japan, the contents of which are incorporated in full herein by this reference.

## Claims

1. A protein having an amino acid sequence shown in SEQ ID NO: 1 or SEQ ID NO: 3, or a variant of said protein, which has an amino acid sequence shown in SEQ ID NO: 1 or SEQ ID NO: 3, wherein one to several amino acid residues are at least substituted, deleted, inserted or added, and has substantially the same level of activity as the cell proliferation activity of said protein, or a fragment thereof.

2. An antibody against the protein of claim 1 or a variant thereof.

3. A polynucleotide molecule encoding the protein of claim 1 or a variant thereof.

4. The polynucleotide molecule of claim 3, which is a DNA molecule.

5. The polynucleotide molecule of claim 3, which is located in the vicinity of human chromosome 5q12, which corresponds to a gene showing upregulated transcription due to binding of c-Myc protein.

6. The polynucleotide molecule of any of claims 3 to 5, which has the 128th - 634th position of SEQ ID NO: 2, or the base sequence shown in SEQ ID NO: 4.

7. A polynucleotide molecule encoding a protein having the base sequence shown in SEQ ID NO: 2 and cell proliferation activity.

8. A polynucleotide molecule having the base sequence shown in SEQ ID NO: 4.

9. A vector containing the polynucleotide molecule of any of claims 3 to 8.

10. A transformant obtained by introducing the vector of claim 9 into a host cell.

11. A method of producing the protein of claim 1, which comprises culturing the transformant of claim 10, and recovering the resulting protein from a culture.

12. A polynucleotide or oligonucleotide molecule having a sequence complementary to a full length or a part of the base sequence shown in SEQ ID NO: 2 or SEQ ID NO: 4, or a variant thereof, which is a molecule inhibiting the cell proliferation activity of the protein of claim 1.

13. The molecule of claim 12, which is an antisense oligonucleotide molecule.

14. The molecule of claim 12, which is a small interfering RNA(siRNA) molecule containing a part of the base sequence shown in SEQ ID NO: 2 or SEQ ID NO: 4, and a complementary chain thereof, which specifically inhibits gene expression.

15. A pharmaceutical composition comprising the molecule of any of claims 12 to 14 as an active ingredient.

16. The pharmaceutical composition of claim 15, which is used for the prophylaxis or treatment of cancer.

17. A diagnostic agent for cancer comprising the antibody of claim 2.

18. A method of detecting the protein of claim 1 in a patient suspected of having a cancer, which comprises labeling a tissue obtained from the patient with the antibody of claim 2, and judging the level of labeling.

19. A method of screening for a substance inhibiting the cell proliferation activity of the polynucleotide molecule of any of claims 3 to 8, which comprises adding a sample containing a candidate inhibitory substance to a reaction system containing the polynucleotide molecule and a c-Myc protein, and determining the activity of the candidate substance to inhibit binding of the polynucleotide molecule and the c-Myc protein.

20. A method for the prophylaxis or treatment of cancer, which comprises administering the pharmaceutical composition of claim 15 or 16 to an animal.

21. A commercial package comprising the pharmaceutical composition of claim 15 or 16 and a written matter associated therewith, the written matter stating that the pharmaceutical composition can or should be used for preventing or treating cancer.


SEQUENCE LISTING

<110> GAKKOUHOUJIN KURUME UNIVERSITY

<120> Myc target gene mimitin

<130> 09761

<150> JP 2004-147964
<151> 2004-05-18

<160> 26

<170> PatentIn Ver. 2.1

<210> 1
<211> 169
<212> PRT
<213> Homo sapiens

<400> 1
Met Gly Trp Ser Gln Asp Leu Phe Arg Ala Leu Trp Arg Ser Leu Ser
  1               5                  10                  15

Arg Glu Val Lys Glu His Val Gly Thr Asp Gln Phe Gly Asn Lys Tyr
             20                  25                  30

Tyr Tyr Ile Pro Gln Tyr Lys Asn Trp Arg Gly Gln Thr Ile Arg Glu
         35                  40                  45

Lys Arg Ile Val Glu Ala Ala Asn Lys Lys Glu Val Asp Tyr Glu Ala
     50                  55                  60

Gly Asp Ile Pro Thr Glu Trp Glu Ala Trp Ile Arg Arg Thr Arg Lys
 65                  70                  75                  80

Thr Pro Pro Thr Met Glu Glu Ile Leu Lys Asn Glu Lys His Arg Glu
                 85                  90                  95

Glu Ile Lys Ile Lys Ser Gln Asp Phe Tyr Glu Lys Glu Lys Leu Leu
            100                 105                 110

Ser Lys Glu Thr Ser Glu Glu Leu Leu Pro Pro Pro Val Gln Thr Gln
        115                 120                 125

Ile Lys Gly His Ala Ser Ala Pro Tyr Phe Gly Lys Glu Glu Pro Ser
    130                 135                 140

Val Ala Pro Ser Ser Thr Gly Lys Thr Phe Gln Pro Gly Ser Trp Met
145                 150                 155                 160

Pro Arg Asp Gly Lys Ser His Asn Gln
                165


<210> 2
<211> 717
<212> DNA
<213> Homo sapiens

<220>
<221> CDS
<222> (128)..(637)

<400> 2
gttactagaa gggctccagg atggaggccg gcctgacttc tccgcctcgg tgggctgggt 60

cggcggctgg agcattaccc ctactgcggg tcccgctgct ggcagcgctg gaaactgggt 120

ggacggc atg ggt tgg tct cag gat ttg ttc cgc gcc ttg tgg aga tcg 169
        Met Gly Trp Ser Gln Asp Leu Phe Arg Ala Leu Trp Arg Ser
          1               5                  10

ctg tca agg gaa gtg aaa gag cac gtg ggc acg gac caa ttc ggg aac 217
Leu Ser Arg Glu Val Lys Glu His Val Gly Thr Asp Gln Phe Gly Asn
 15                  20                  25                  30

aaa tac tac tac atc ccg cag tac aag aac tgg aga gga caa act att 265
Lys Tyr Tyr Tyr Ile Pro Gln Tyr Lys Asn Trp Arg Gly Gln Thr Ile
                 35                  40                  45

cga gag aaa aga att gta gaa gca gca aat aaa aaa gaa gta gac tat 313
Arg Glu Lys Arg Ile Val Glu Ala Ala Asn Lys Lys Glu Val Asp Tyr
             50                  55                  60

gaa gca ggg gat att cca aca gaa tgg gaa gct tgg att aga aga aca 361
Glu Ala Gly Asp Ile Pro Thr Glu Trp Glu Ala Trp Ile Arg Arg Thr
         65                  70                  75

aga aag act cca cct act atg gag gaa ata cta aag aat gaa aaa cac 409
Arg Lys Thr Pro Pro Thr Met Glu Glu Ile Leu Lys Asn Glu Lys His
     80                  85                  90

aga gaa gaa atc aaa ata aaa agc caa gat ttt tat gaa aaa gaa aaa 457
Arg Glu Glu Ile Lys Ile Lys Ser Gln Asp Phe Tyr Glu Lys Glu Lys
 95                 100                 105                 110

ctc ctt agt aaa gag acc agt gag gaa ctc ctg cct cca cca gtt caa 505
Leu Leu Ser Lys Glu Thr Ser Glu Glu Leu Leu Pro Pro Pro Val Gln
                115                 120                 125

act caa att aaa ggc cat gcc tct gct cca tac ttt gga aag gaa gaa 553
Thr Gln Ile Lys Gly His Ala Ser Ala Pro Tyr Phe Gly Lys Glu Glu
            130                 135                 140

ccc tca gtg gct ccc agc agc act ggt aaa acc ttt cag cca gga tcc 601
Pro Ser Val Ala Pro Ser Ser Thr Gly Lys Thr Phe Gln Pro Gly Ser
        145                 150                 155


tgg atg cca cga gat ggc aag agc cac aat caa tga atgcattatg 647
Trp Met Pro Arg Asp Gly Lys Ser His Asn Gln
    160                 165                 170

gtcaaatctt ttcatgtata tggatgtgac tattttaaca aataaaagaa gtgaaaagtt 707

aaaaaaaaaa 717



<210> 3
<211> 168
<212> PRT
<213> Mus musculus

<400> 3

Met Ser Trp Trp Ser Gly Val Trp Arg Ser Val Trp Ser Ala Leu Ser
1               5                   10                  15

Arg Glu Val Arg Glu His Val Gly Thr Asp His Leu Gly Asn Lys Tyr
            20                  25                  30

Tyr Tyr Val Ala Glu Tyr Lys Asn Trp Arg Gly Gln Thr Ile Arg Glu
        35                  40                  45

Lys Arg Ile Val Glu Ala Ala Asn Arg Lys Glu Val Asp Tyr Glu Ala
    50                  55                  60

Gly Asp Ile Pro Thr Glu Trp Glu Ala Trp Ile Arg Arg Thr Arg Lys
65                  70                  75                  80

Thr Pro Pro Thr Met Glu Glu Ile Leu Lys Asn Glu Lys Tyr Arg Glu
                85                  90                  95

Glu Ile Lys Ile Lys Ser Gln Asp Phe Tyr Glu Lys Asp Lys Leu Gly
            100                 105                 110

Lys Glu Thr Ser Glu Glu Leu Leu Pro Ser Pro Thr Ala Thr Gln Val
        115                 120                 125

Lys Gly His Ala Ser Ala Pro Tyr Phe Gly Arg Glu Glu Pro Ser Val
    130                 135                 140

Ala Pro Thr Ser Thr Gly Lys Thr Phe Gln Pro Gly Ser Trp Thr Pro
145                 150                 155                 160

Glu Asp Gly Lys Arg Gln Ser Gln
                165


<210> 4
<211> 507
<212> DNA
<213> Mus musculus

<400> 4
atgagctggt ggtccggtgt gtggcgctct gtttggagcg ccttgtccag ggaggtgagg 60
gagcacgtgg gcacggacca tctggggaac aaatactact acgtcgccga gtacaagaac 120
tggagagggc agactattcg agagaaaagg attgtagaag cagcgaacag aaaagaggta 180
gactatgaag caggagacat cccaaccgag tgggaagcat ggattagaag aacaaggaag 240
actccaccca ctatggagga aatactaaag aatgaaaagt acagagaaga aattaaaata 300
aaaagccaag atttttatga aaaagataaa ctcggtaaag aaaccagtga agaactcttg 360
ccttcaccta ctgcaactca agttaaaggg cacgcctctg ccccgtattt tggaagggaa 420
gagccctcag tggctcccac cagcactggc aagacttttc agccagggtc ctggacgcca 480
gaagatggca aaagacaaag tcaatga                                     507


<210> 5
<211> 25
<212> DNA
<213> Artificial Sequence

<220>
<223> PCR primer

<400> 5
gctgctggga gccactgagg gttct                                       25

<210> 6
<211> 27
<212> DNA
<213> Artificial Sequence

<220>
<223> PCR primer

<400> 6
ccatcctaat acgactcact atagggc                                          27


<210> 7
<211> 26
<212> DNA
<213> Artificial Sequence

<220>
<223> PCR primer


<400> 7
gcatggcctt taatttgagt ttgaac                                           26


<210> 8
<211> 23
<212> DNA
<213> Artificial Sequence

<220>
<223> PCR primer

<400> 8
actcactata gggctcgagc ggc                                              23


<210> 9
<211> 25
<212> DNA
<213> Artificial Sequence

<220>
<223> PCR primer


<400> 9
gcggctggag cattacccct actgc                                            25


<210> 10
<211> 25
<212> DNA
<213> Artificial Sequence

<220>
<223> PCR Primer

<400> 10
aaactgggtg gacggcatgg gttgg                                            25


<210> 11

<211> 38
<212> DNA
<213> Artificial Sequence

<220>
<223> PCR primer

<400> 11
ccggaattct gccatgggtt ggtctcagga tttgttcc                                38


<210> 12
<211> 40
<212> DNA
<213> Artificial Sequence

<220>
<223> PCR Primer

<400> 12
ggaagatctg aattcattga ttgtggctct tgccatctcg                              40


<210> 13
<211> 44
<212> DNA
<213> Artificial Sequence

<220>
<223> PCR Primer

<400> 13
ccggaattca ggcaggcatg agctggtggt ccggtgtgtg gcgc                         44


<210> 14
<211> 35
<212> DNA
<213> Artificial Sequence

<220>
<223> PCR Primer

<400> 14
ggtcgaattc attgactttg tcttttgcca tcttc                                   35



<210> 15
<211> 26
<212> DNA
<213> Artificial Sequence

<220>
<223> PCR Primer

<400> 15
tgaaggtcgg agtcaacgga tttggt                                             26


<210> 16
<211> 24
<212> DNA

<213> Artificial Sequence

<220>
<223> PCR Primer

<400> 16
catgtgggcc atgaggtcca ccac                                              24


<210> 17
<211> 24
<212> DNA
<213> Artificial Sequence

<220>
<223> PCR Primer

<400> 17
gtggagggca gttattcttt ggag                                              24


<210> 18
<211> 24
<212> DNA
<213> Artificial Sequence

<220>
<223> PCR Primer

<400> 18
gggctctcac ttttccttcc ttta                                              24


<210> 19
<211> 32
<212> DNA
<213> Artificial Sequence

<220>
<223> PCR Primer

<400> 19
gtggagacgc gttattcttt ggagtaactt aa                                     32


<210> 20
<211> 33
<212> DNA
<213> Artificial Sequence

<220>
<223> PCR Primer

<400> 20
ggggctccca tggttccttc ctttactgac ctc                                    33


<210> 21
<211> 24
<212> DNA
<213> Artificial Sequence

<220>

<223> PCR Primer

<400> 21
ttacaggtaa gccctccaat gacc                                              24


<210> 22
<211> 23
<212> DNA
<213> Artificial Sequence

<220>
<223> PCR Primer

<400> 22
gcaaagctac catttaggaa ccc                                               23


<210> 23
<211> 21
<212> RNA
<213> Artificial Sequence

<220>
<223> siRNA

<220>
<221> modified_base
<222> (20)..(21)
<223> n=dT

<400> 23
cacggaccaa uucgggaacn n                                                 21


<210> 24
<211> 21
<212> RNA
<213> Artificial Sequence

<220>
<221> modified_base
<222> (20)..(21)
<223> siRNA

<220>
<223> n=dT

<400> 24
guucccgaau ugguccgugn n                                                 21


<210> 25
<211> 21
<212> RNA
<213> Artificial Sequence

<220>
<223> siRNA

<220>
<221> modified_base
<222> (20)..(21)

<223> n=dT

<400> 25
acuccuuagu aaagagaccn n                                                21


<210> 26
<211> 21
<212> RNA
<213> Artificial Sequence

<220>
<223> siRNA

<220>
<221> modified_base
<222> (20)..(21)
<223> n=dT

<400> 26
ggucucuuua cuaaggagun n                                                21